# EUROPEAN PATENT APPLICATION

(11) **EP 4 008 774 A1**
(43) Date of publication of application: **08.06.2022**
(21) Application number: 20840317.0
(22) Date of filing: 16.07.2020
(51) Int. Cl.: C12N 5/02

(54) **CELL CULTURE METHOD AND APPLICATION THEREOF BASED ON HIGH-DENSITY AND CONTINUOUS INOCULATION**

(30) Priority: 16.07.2019 CN 201910641684
(71) Applicant: Innovent Biologics (Suzhou) Co., Ltd., Suzhou, Jiangsu 215123 (CN)
(72) Inventor: SUN, Haihong, Suzhou, Jiangsu 215123 (CN); ZHAO, Xizhang, Suzhou, Jiangsu 215123 (CN); JIANG, Qingyi, Suzhou, Jiangsu 215123 (CN); SHI, Zhengpeng, Suzhou, Jiangsu 215123 (CN); LI, Jianfeng, Suzhou, Jiangsu 215123 (CN); ZHOU, Kaisong, Suzhou, Jiangsu 215123 (CN)
(74) Representative: Nederlandsch Octrooibureau
(86) International application number: PCT/CN2020/102258
(87) International publication number: WO 2021/008571

(57) **Abstract**

The present invention discloses a cell culture method based on high-density and continuous inoculation and use thereof. The method comprises the following steps: (1) providing a cell culture, and performing resuscitation, shake flask amplification culture and rocking reaction bag amplification culture on the cell culture; (2) transferring the resuscitated and amplified cells into a last-stage cell amplification tank for continuous amplification culture; (3) inoculating the cells in the last-stage cell amplification tank into a culture fermentation tank in a high-density and continuous inoculation mode for fermentation culture; and (4) harvesting a target product. The cell culture method based on high-density and continuous inoculation provided herein can reduce the average cell passage time of each batch and improve the production efficiency of cells and expression products thereof.

SEQUENCE LISTING

<110>  Innovent Biologics (Suzhou) Co., Ltd.

<120>  Cell Culture Method Based on High-Density and Continuous
       Inoculation and Use thereof

<130>  6722-190608EP

<150>  201910641684.6
<151>  2019-07-16

<160>  4

<170>  PatentIn version 3.5

<210>  1
<211>  446
<212>  PRT
<213>  Artificial sequence

<220>
<223>  Synthetic Construct

<400>  1

Gln Leu Gln Leu Gln Glu Ser Gly Pro Gly Leu Val Lys Pro Ser Glu
1               5                   10                  15

Thr Leu Ser Leu Thr Cys Thr Val Ser Gly Gly Ser Ile Tyr Ser Glu
            20                  25                  30

Ser Tyr Tyr Trp Gly Trp Ile Arg Gln Pro Pro Gly Lys Gly Leu Glu
        35                  40                  45

Trp Ile Gly Ser Ile Val Tyr Ser Gly Tyr Thr Tyr Tyr Asn Pro Ser
    50                  55                  60

Leu Lys Ser Arg Val Thr Ile Ser Val Asp Thr Ser Lys Asn Gln Phe
65                  70                  75                  80

Ser Leu Lys Leu Ser Ser Val Thr Ala Ala Asp Thr Ala Val Tyr Tyr
                85                  90                  95

Cys Ala Arg Val Arg Thr Trp Asp Ala Ala Phe Asp Ile Trp Gly Gln
            100                 105                 110

Gly Thr Met Val Thr Val Ser Ser Ala Ser Thr Lys Gly Pro Ser Val
        115                 120                 125

Phe Pro Leu Ala Pro Cys Ser Arg Ser Thr Ser Glu Ser Thr Ala Ala
    130                 135                 140

Leu Gly Cys Leu Val Lys Asp Tyr Phe Pro Glu Pro Val Thr Val Ser

145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Lys Thr Tyr Thr Cys Asn Val Asp His Lys
        195                 200                 205

Pro Ser Asn Thr Lys Val Asp Lys Arg Val Glu Ser Lys Tyr Gly Pro
    210                 215                 220

Pro Cys Pro Pro Cys Pro Ala Pro Glu Ala Ala Gly Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Asp Val Ser Gln Glu Asp Pro Glu
            260                 265                 270

Val Gln Phe Asn Trp Tyr Val Asp Gly Val Glu Val His Asn Ala Lys
        275                 280                 285

Thr Lys Pro Arg Glu Glu Gln Phe Asn Ser Thr Tyr Arg Val Val Ser
    290                 295                 300

Val Leu Thr Val Leu His Gln Asp Trp Leu Asn Gly Lys Glu Tyr Lys
305                 310                 315                 320

Cys Lys Val Ser Asn Lys Gly Leu Pro Ser Ser Ile Glu Lys Thr Ile
                325                 330                 335

Ser Lys Ala Lys Gly Gln Pro Arg Glu Pro Gln Val Tyr Thr Leu Pro
            340                 345                 350

Pro Ser Gln Glu Glu Met Thr Lys Asn Gln Val Ser Leu Thr Cys Leu
        355                 360                 365

Val Lys Gly Phe Tyr Pro Ser Asp Ile Ala Val Glu Trp Glu Ser Asn
    370                 375                 380

Gly Gln Pro Glu Asn Asn Tyr Lys Thr Thr Pro Pro Val Leu Asp Ser
385                 390                 395                 400

Asp Gly Ser Phe Phe Leu Tyr Ser Arg Leu Thr Val Asp Lys Ser Arg
                405                 410                 415

Trp Gln Glu Gly Asn Val Phe Ser Cys Ser Val Met His Glu Ala Leu
            420                 425                 430

His Asn His Tyr Thr Gln Lys Ser Leu Ser Leu Ser Leu Gly
        435                 440                 445

<210> 2
<211> 215
<212> PRT
<213> Artificial sequence

<220>
<223> Synthetic Construct

<400> 2

Asp Ile Gln Met Thr Gln Ser Pro Ser Ser Leu Ser Ala Ser Val Gly
1               5                   10                  15

Asp Arg Val Thr Ile Thr Cys Gln Ala Ser Gln Asp Ile Ser Asn Tyr
            20                  25                  30

Leu Asn Trp Tyr Gln Gln Lys Pro Gly Lys Ala Pro Lys Leu Leu Ile
        35                  40                  45

Tyr Asp Ala Ser Asn Leu Glu Thr Gly Val Pro Ser Arg Phe Ser Gly
    50                  55                  60

Ser Gly Ser Gly Thr Asp Phe Thr Phe Thr Ile Ser Ser Leu Gln Pro
65                  70                  75                  80

Glu Asp Ile Ala Thr Tyr Tyr Cys Gln Gln Val Leu Glu Leu Pro Pro
                85                  90                  95

Trp Thr Phe Gly Gly Gly Thr Lys Val Glu Ile Lys Arg Thr Val Ala
            100                 105                 110

Ala Pro Ser Val Phe Ile Phe Pro Pro Ser Asp Glu Gln Leu Lys Ser
        115                 120                 125

Gly Thr Ala Ser Val Val Cys Leu Leu Asn Asn Phe Tyr Pro Arg Glu
    130                 135                 140

Ala Lys Val Gln Trp Lys Val Asp Asn Ala Leu Gln Ser Gly Asn Ser
145                 150                 155                 160

Gln Glu Ser Val Thr Glu Gln Asp Ser Lys Asp Ser Thr Tyr Ser Leu

                165                 170                 175

Ser Ser Thr Leu Thr Leu Ser Lys Ala Asp Tyr Glu Lys His Lys Val
            180                 185                 190

Tyr Ala Cys Glu Val Thr His Gln Gly Leu Ser Ser Pro Val Thr Lys
        195                 200                 205

Ser Phe Asn Arg Gly Glu Cys
    210                 215

<210> 3
<211> 591
<212> PRT
<213> Artificial sequence

<220>
<223> Synthetic Construct

<400> 3

Gln Leu Gln Leu Gln Glu Ser Gly Pro Gly Leu Val Lys Pro Ser Glu
1               5                   10                  15

Thr Leu Ser Leu Thr Cys Thr Val Ser Gly Gly Ser Ile Tyr Ser Glu
            20                  25                  30

Ser Tyr Tyr Trp Gly Trp Ile Arg Gln Pro Pro Gly Lys Gly Leu Glu
        35                  40                  45

Trp Ile Gly Ser Ile Val Tyr Ser Gly Tyr Thr Tyr Tyr Asn Pro Ser
    50                  55                  60

Leu Lys Ser Arg Val Thr Ile Ser Val Asp Thr Ser Lys Asn Gln Phe
65                  70                  75                  80

Ser Leu Lys Leu Ser Ser Val Thr Ala Ala Asp Thr Ala Val Tyr Tyr
                85                  90                  95

Cys Ala Arg Val Arg Thr Trp Asp Ala Ala Phe Asp Ile Trp Gly Gln
            100                 105                 110

Gly Thr Met Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Lys Val Glu Pro Lys Ser Cys Asp
    210                 215                 220

Lys Thr His Thr Cys Pro Pro Cys Pro Ala Pro Glu Ala Ala Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asn Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Ala Leu Pro Ala Pro Ile Glu
                325                 330                 335

Lys Thr Ile Ser Lys Ala Lys Gly Gln Pro Arg Glu Pro Gln Val Tyr
            340                 345                 350

Thr Leu Pro Pro Ser Arg Asp Glu Leu Thr Lys Asn Gln Val Ser Leu
        355                 360                 365

Thr Cys Leu Val Lys Gly Phe Tyr Pro Ser Asp Ile Ala Val Glu Trp
    370                 375                 380

Glu Ser Asn Gly Gln Pro Glu Asn Asn Tyr Lys Thr Thr Pro Pro Val
385                 390                 395                 400

Leu Asp Ser Asp Gly Ser Phe Phe Leu Tyr Ser Lys Leu Thr Val Asp

405                 410                 415

Lys Ser Arg Trp Gln Gln Gly Asn Val Phe Ser Cys Ser Val Met His
            420                 425                 430

Glu Ala Leu His Asn His Tyr Thr Gln Lys Ser Leu Ser Leu Ser Pro
        435                 440                 445

Gly Gly Gly Gly Gly Ser Gly Gly Gly Gly Ser Gln Val Gln Leu Gln
    450                 455                 460

Glu Ser Gly Gly Gly Leu Val Gln Pro Gly Gly Ser Leu Arg Leu Ser
465                 470                 475                 480

Cys Ala Ala Ser Ala Tyr Thr Ile Ser Arg Asn Ser Met Gly Trp Phe
                485                 490                 495

Arg Gln Ala Pro Gly Lys Gly Leu Glu Gly Val Ala Ala Ile Glu Ser
            500                 505                 510

Asp Gly Ser Thr Ser Tyr Ser Asp Ser Val Lys Gly Arg Phe Thr Ile
        515                 520                 525

Ser Leu Asp Asn Ser Lys Asn Thr Leu Tyr Leu Glu Met Asn Ser Leu
    530                 535                 540

Arg Ala Glu Asp Thr Ala Val Tyr Tyr Cys Ala Ala Pro Lys Val Gly
545                 550                 555                 560

Leu Gly Pro Arg Thr Ala Leu Gly His Leu Ala Phe Met Thr Leu Pro
                565                 570                 575

Ala Leu Asn Tyr Trp Gly Gln Gly Thr Leu Val Thr Val Ser Ser
            580                 585                 590

<210>  4
<211>  215
<212>  PRT
<213>  Artificial sequence

<220>
<223>  Synthetic Construct

<400>  4

Asp Ile Gln Met Thr Gln Ser Pro Ser Ser Leu Ser Ala Ser Val Gly
1               5                   10                  15

Asp Arg Val Thr Ile Thr Cys Gln Ala Ser Gln Asp Ile Ser Asn Tyr
            20                  25                  30

Leu Asn Trp Tyr Gln Gln Lys Pro Gly Lys Ala Pro Lys Leu Leu Ile
        35                  40                  45

Tyr Asp Ala Ser Asn Leu Glu Thr Gly Val Pro Ser Arg Phe Ser Gly
    50                  55                  60

Ser Gly Ser Gly Thr Asp Phe Thr Phe Thr Ile Ser Ser Leu Gln Pro
65                  70                  75                  80

Glu Asp Ile Ala Thr Tyr Tyr Cys Gln Gln Val Leu Glu Leu Pro Pro
                85                  90                  95

Trp Thr Phe Gly Gly Gly Thr Lys Val Glu Ile Lys Arg Thr Val Ala
            100                 105                 110

Ala Pro Ser Val Phe Ile Phe Pro Pro Ser Asp Glu Gln Leu Lys Ser
        115                 120                 125

Gly Thr Ala Ser Val Val Cys Leu Leu Asn Asn Phe Tyr Pro Arg Glu
    130                 135                 140

Ala Lys Val Gln Trp Lys Val Asp Asn Ala Leu Gln Ser Gly Asn Ser
145                 150                 155                 160

Gln Glu Ser Val Thr Glu Gln Asp Ser Lys Asp Ser Thr Tyr Ser Leu
                165                 170                 175

Ser Ser Thr Leu Thr Leu Ser Lys Ala Asp Tyr Glu Lys His Lys Val
            180                 185                 190

Tyr Ala Cys Glu Val Thr His Gln Gly Leu Ser Ser Pro Val Thr Lys
        195                 200                 205

Ser Phe Asn Arg Gly Glu Cys
    210                 215

## Description

### PRIORITY AND RELATED APPLICATION

The present application claims priority to Chinese patent application No. 201910641684.6 entitled "CELL CULTURE METHOD BASED ON HIGH-DENSITY AND CONTINUOUS INOCULATION AND USE THEREOF" and filed on Jul. 16, 2019, which is incorporated herein by reference in its entirety together with the appendix.

### TECHNICAL FIELD

The present invention relates to the technical field of mammalian cell culture, and in particular to a cell culture method based on high-density and continuous inoculation and use thereof.

### BACKGROUND

Over decades of development, cell culture technique has become a mature technique for the production of many important proteins, especially complex macromolecular proteins. These proteins are often used in the prevention and treatment of several serious human diseases, such as cancer, viral infections, genetic defect diseases and other chronic diseases.

At present, the *in vitro* cell culture model mainly comprises three types: batch culture, fed-batch culture and perfusion culture. The batch culture refers to a culture mode that a certain amount of culture medium is put into a closed reactor and then microbial strains are inoculated for culture. The fed-batch culture refers to a culture mode that a certain amount of culture solution is firstly filled into a reactor, cells are inoculated under appropriate conditions for culture, the cells grow continuously with products being formed continuously, and in this process, new nutrient components are continuously supplemented into a system along with the continuous consumption of nutrient substances, the cells are further grown and metabolized, and finally the products are taken out until the whole culture process is finished. The fed-batch culture is characterized in that the concentration of nutrient substances in the culture environment can be adjusted, and meanwhile, in the fed-batch culture process, the reaction volume in the whole process varies due to the addition of a fresh culture solution. Compared with batch culture and fed-batch culture, the perfusion culture (or called open culture or perfused culture) is increasingly appreciated and popularized because of its unique advantages, such as continuous production, high cultured cell density and good consistency of final products.

The perfusion culture is a culture method which is preferred in recent years for the production of secretory recombinant therapeutic drugs such as monoclonal antibodies, chimeric antibodies, humanized antibodies and other genetically engineered antibodies in mammalian cell culture. The perfusion culture refers to a process of continuously removing part of the conditioned culture medium and simultaneously continuously perfusing new culture medium during the process of cell growth and product formation after the cells and medium are added together into the reactor. It has the following advantages: (1) the cells can be in a culture environment with rich nutrition, and the concentration of harmful metabolic waste is low; (2) the cell density is high and can reach 10⁷-10⁸ cells/mL generally, thereby greatly improving the yield of target products (such as monoclonal antibodies); (3) the method has high cell density and long survival time, so the recovery rate of target products is high; (4) the target products have short retention time in the culture fermentation tank, can be timely recovered to be stored at low temperature, and thus are beneficial to maintaining their activity.

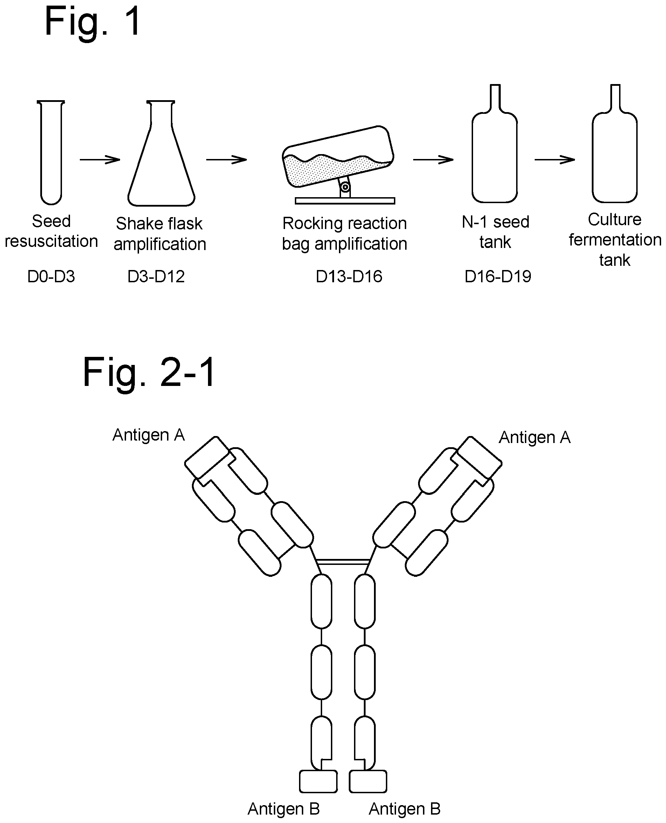

Continuous staged culture using shake flasks and reactors is a typical cell amplification process. When the cell culture volume and the cell density reach an expected value, cells are inoculated into a culture fermentation tank for culture and expression. In the conventional process (see FIG. 1), about 19 days are generally required from the beginning of resuscitation of cells to the inoculation of the cells into the culture fermentation tank (D0-D19), and about 15 days are required for production (namely about 15 days are required for production in the culture fermentation tank), so the total cell culture time is about 34 days. The cells take about 7 days to reach an appropriate density in the culture fermentation tank, and then enormous target proteins are to be produced. Thus, the first 7 days can be called a growth phase and the last 8 days an expression phase. If an inoculation in the culture fermentation tank is performed at an inoculation density 10 times higher than a normal inoculation density, the growth phase can be shortened considerably, allowing the cells to reach the previous 15 days of yields in a shorter time. This technique is known as high-density inoculation. However, in the biopharmaceutical field, the high-density inoculation technique is not widely used, and the most important reason is that a conventional last-stage cell amplification tank (also called N-1 seed tank) cannot provide such a large amount of cells.

CN108641960A discloses a multipurpose bioreactor, which comprises a culture tank, a stirring mechanism, a ventilation mechanism, a feeding mechanism, a harvesting device and an exhaust device, wherein related module components can be replaced in the bioreactor according to different cultured cells, so as to meet culture requirements of various cells, and thus the bioreactor disclosed herein can be widely used for large-scale high-density cell culture and target product expression in the biopharmaceutical industry. CN108949558A discloses a drum hollow fiber reactor for high-density culture of plant cells, wherein the reactor has good mixing performance, low shearing force and energy conservation during cell culture; in addition, the rotation of a drum drives a fluid in a shell cavity to flow, so that the compact agglomeration and clumping of cells can be greatly reduced, the concentration of dissolved oxygen in the cells can be increased, the mass transfer permeability of membranes can be improved, the growth and metabolism of the cells can be facilitated, and thus the high-density cell culture can be facilitated. CN109576212A discloses a seed cell culture method based on high-density inoculation and culture, wherein the method increases the viable cell density by fed-batch culture after N-1 seed culture and in subsequent amplification culture. In the fed-batch culture method described in this document, the total amount of substances increases, metabolites and target proteins expressed by cells accumulate, cell debris increases, and an optimum cell culture environment cannot be maintained, so that it is difficult to increase the density of a seed culture medium.

Under large-scale production conditions (typically greater than 1000 L), a culture fermentation tank requires a quantity of cells to be the starting "seeds", and the cells will be transferred into a bioreactor for continuous growth and product expression when the cell culture volume and the cell density meet predetermined criteria. And this part of cells need to be amplified in multiple stages and steps to reach a necessary density requirement. In the conventional process, the cell resuscitation is started, and then the cells are amplified through continuous subculture, wherein the selected culture equipment comprises a shake flask, a WAVE bioreactor, a stirring reactor, and the like. The conventional process, when selected for cell culture, has the disadvantages of long time consumption and low efficiency in a cell amplification stage. Therefore, with an increasing demand of the future market on biological products, the establishment of a novel efficient and time-saving amplification process is urgent.

### SUMMARY

### Problems to be solved herein

Aiming at the problems in the prior art, the present application provides a cell culture method based on high-density and continuous inoculation capable of reducing the average cell passage time of each batch and improving the production efficiency of cells and expression products thereof, and use thereof.

### Solutions for solving the problems

In view of the problems of the prior art described above, the inventors have made extensive studies and experiments, and have accomplished the present invention by the optimization of the cell amplification process and shortening the average cell passage time of each batch. Thus, the present invention is as follows:
In a first aspect of the present invention, provided is a cell culture method based on high-density and continuous inoculation, comprising the following steps:
(1) providing a cell culture, and performing resuscitation, shake flask amplification culture and rocking reaction bag amplification culture on the cell culture;
(2) transferring the resuscitated and amplified cells into a last-stage cell amplification tank for continuous amplification culture;
(3) inoculating the cells in the last-stage cell amplification tank into a culture fermentation tank in a high-density and continuous inoculation mode for fermentation culture; and
(4) harvesting a target product.

In the above method, the last-stage cell amplification tank in step (2) is provided with a filtration device, and non-cellular materials in the culture medium can be exchanged with a fresh culture medium by using the filtration device, so that a cell density in the last-stage cell amplification tank is more than 10⁷ cells/mL; preferably, the filtration device is ATF, Spin filter or TFF.

Furthermore, the high-density and continuous inoculation in step (3) is to inoculate the cells into the culture fermentation tank at a density of more than 10⁷ cells/mL. In a specific embodiment of the present invention, the high-density and continuous inoculation in step (3) is to inoculate a cell culture solution with a volume less than or equal to half the volume in the last-stage cell amplification tank into the culture fermentation tank at the density of more than 10⁷ cells/mL, and after the inoculation is completed, the last-stage cell amplification tank is supplemented with the same volume of fresh culture medium, so that the fresh culture medium can be inoculated into another new culture fermentation tank the next day after inoculation; the above operations of inoculating the cell culture solution into the culture fermentation tank and supplementing the fresh culture medium in the last-stage cell amplification tank are repeated.

Specifically, an early-stage passage process of the method provided herein is consistent with a conventional method, but at most half of the cell culture solution is inoculated into the culture fermentation tank after the cells are cultured in the N-1 seed tank for 3 days, and then the fresh culture medium with the same volume is supplemented; the multiplication time of CHO cells is about 24 hours, so that the seed cells with similar density can be obtained by adopting the method disclosed herein every other day and used for inoculation in a new culture fermentation tank, and so on. According to the maximum culture days (B) specified by the cell stability data, the N-1 seed tank can realize continuous culture and continuous inoculation within a certain time (< B), so that the time and cost can be greatly saved. The cell culture method based on high-density and continuous inoculation provided herein can adopt a perfusion culture process to improve the seed density of an N-1 seed tank followed by inoculation.

In a second aspect of the present invention, the cell culture method based on high-density and continuous inoculation in the first aspect is improved by performing the resuscitation, the shake flask amplification culture and the rocking reaction bag amplification culture in step (1) on the cell culture in two batches, and adding another last-stage cell amplification tank in step (2).

Specifically, in order to further solve the defect that the maximum culture days of the N-1 seed tank in the technical solution provided in the first aspect of the present invention are limited by insufficient cell stability, after one batch of cells are resuscitated for C days (C is less than the maximum days specified by the passage stability), a second batch of cells are resuscitated, and the cells can be placed in the tank after being cultured in the N-1 seed tank for C days according to the culture method provided in the first aspect of the present invention. At this point, the second batch of seeds are cultured in the N-1 seed tank and ready for continuous inoculation in the culture fermentation tank. The culture method can also adopt a perfusion culture process to improve the seed density of an N-1 seed tank followed by inoculation.

The cell culture method based on high-density and continuous inoculation provided in the first and second aspects of the present invention is characterized in that a volume of the last-stage cell amplification tank in step (2) is 2 L to 1000 L.

In a specific embodiment, the heating type of the reactor tank having a volume of 2-10 L used in the method provided herein is glass tank heating mat heating, wherein a bottom ventilation end is annular small bubbles or straight-through micro bubbles; the heating type of the reactor tank having a volume of 250 L and 1000 L is stainless steel tank water jacket heating, wherein a culture solution is filled in a disposable culture bag and not in contact with the stainless steel tank. The reactor tank of each specification is a commercialized third-party product. When the method provided herein is applied to actual production in a factory, a culture vessel having a volume of 1000 L or more, for example, a disposable reaction bag having a volume of 1000 L or a stainless steel reactor having a volume of 3000 L or more, may be used.

In a third aspect of the present invention, provided is use of the cell culture method based on high-density and continuous inoculation described above for the culture of mammalian cells.

The mammalian cells are selected from the group consisting of CHO, DXB-11, DG-44, CHO/-DHFR, CV1, COS-7, HEK293, BHK, TM4, VERO, HELA, MDCK, BRL3A, W138, Hep G2, SK-Hep, MMT, TRI, MRC5, FS4, T cell lines, B cell lines, 3T3, RIN, A549, PC12, K562, PER.C6, SP2/0, NS-0, U20S, HT1080, L929, hybridomas and cancer cell lines. Preferably, the mammalian cells are CHO-S cell lines of Chinese hamster ovary cells or CHO-K1 cells of a GS deficient expression system.

In a specific embodiment, the mammalian cells comprise a nucleotide sequence encoding a foreign protein; preferably, the foreign protein is an antibody; more preferably, the antibody is a monoclonal antibody or a bispecific antibody.

### Beneficial effects

As can be seen from the test results of the present invention, compared with the prior art, the technical solution of the present invention has the following beneficial effects:
1. In the present invention, the cell amplification method in the cell amplification tank is improved on the basis of the conventional process, and particularly, the cell amplification method in the last-stage cell amplification tank (namely the N-1 seed tank) is optimized; with the use of a continuous culture technique, the N-1 seed tank can reach a high density so as to obtain enough cells, and the cells are inoculated into the culture fermentation tank at a high density, thereby shortening the production period and reducing the cost. In the conventional cell inoculation technique, cells have a low density (generally not exceeding 10⁶ cells/mL), and about 7 days after inoculation are required for reaching a highest density and then entering a high-yield stage. With the use of the high-density inoculation technology disclosed herein (exceeding 10⁷ cells/mL), cells can reach a high-yield density more quickly, so that the production time is greatly shortened, and the production efficiency is improved.
2. In the present invention, the N-1 seed tank is supplemented with a fresh culture medium to achieve continuous inoculation of the medium into another culture fermentation tank the next day after inoculation. For the second culture fermentation tank, the cell amplification time is only 1 day, so that the cell culture period is greatly shortened compared with the conventional cell culture method (about 19 days are needed from the resuscitation of seeds to the inoculation into the culture fermentation tank). According to the method of the present invention, 6 culture fermentation tanks are inoculated with an N-1 seed tank for 6 consecutive days, and under the condition of the same total yield, a total seed amplification time of 90 days and a production time of 42 days can be saved. And because one N-1 seed tank can achieve the task of continuous inoculation of culture fermentation tanks for multiple days, theoretically, the method can achieve the production efficiency of one batch per day, and finally achieve the purpose of harvesting one batch per day. Therefore, the production efficiency can be greatly improved, and the cost can be reduced.
3. In the present invention, a continuous culture mode is adopted to be applied to the cell amplification of the N-1 seed tank, and non-cellular materials such as metabolites, cell debris and produced protein in the continuous culture environment are continuously exchanged with a fresh culture medium through a filtration device (such as ATF, Spin filter or TFF), thereby maintaining an optimum cell culture environment and improving the density of the seed culture medium.
4. By addition of another N-1 seed tank, the method provided herein is also suitable for the culture of host cells with poor stability and the expression of target proteins.
5. The reactor tank of each specification (2-1000 L) used in the method provided herein is a commercialized third-party product, and the culture method is convenient to implement in laboratories and production workshops.

In order to make the aforementioned and other objectives, features and advantages of the present invention comprehensible, preferred embodiments accompanied with figures are described in detail below.

### BRIEF DESCRIPTON OF THE DRAWINGS

FIG. 1 shows a conventional process for cell culture.
FIG. 2-1 and FIG. 2-2 show a schematic structural diagram and a detailed structural diagram of the INGLP bispecific antibody, respectively.
FIG. 3 shows a graph of the cell viability (VIB) and the viable cell density (VCD) of CHO cells capable of stably expressing the ADI-31853 monoclonal antibody for seed amplification in a 2 L Applikon reactor using ATF perfusion equipment over time.
FIG. 4 shows a graph of the viable cell density of CHO cells stably expressing the ADI-31853 monoclonal antibody for a high-density inoculation (20 × 10⁶ cells/mL, abbreviated as "20 inoculation") and for a normal fed-batch culture inoculation over time.
FIG. 5 shows a graph of the expression yield of the ADI-31853 monoclonal antibody expressed by CHO cells for a high-density inoculation (20 × 10⁶ cells/mL, abbreviated as "20 inoculation") and for a normal fed-batch culture inoculation over time.
FIG. 6 shows a graph of the cell viability (VIB) and the viable cell density (VCD) of CHO seed cells capable of stably expressing the INGLP bispecific antibody for seed amplification in a 10 L culture bag using a Sartorius RM20 reactor over time.
FIG. 7 shows a graph of the viable cell density of CHO cells stably expressing the INGLP bispecific antibody for two different high-density inoculations (10 × 10⁶ cells/mL and 20 × 10⁶ cells/mL, respectively, abbreviated as "10 inoculation" and "20 inoculation", respectively) and for a normal fed-batch culture inoculation over time.
FIG. 8 shows a graph of the expression yield of the INGLP bispecific antibody expressed by CHO cells for two different high-density inoculations (10 × 10⁶ cells/mL and 20 × 10⁶ cells/mL, respectively, abbreviated as "10 inoculation" and "20 inoculation", respectively) and for a normal fed-batch culture inoculation over time.

### DETAILED DESCRIPTION

The technical solution of the present invention is further illustrated with reference to the following specific embodiments. It should be emphasized that the present invention is not limited to the specific embodiments listed herein. Moreover, any section headings used herein are for organizational purposes only and are not to be construed as limiting the subject matter described.

Unless otherwise defined herein, scientific and technical terms used in connection with the present invention will have the meanings that are commonly understood by those of ordinary skill in the art. Furthermore, unless otherwise required in the context, terms used in the singular form shall include the plural form, and vice versa. More specifically, as used in this specification and the appended claims, unless otherwise clearly defined in the context, the singular forms "a", "an" and "the" include referents in the plural form. In the present application, unless otherwise specified, the use of "or" means "and/or". Furthermore, the use of the term "comprise" or "comprising" and other forms such as "include", "including" or "containing" is not limiting. Moreover, the ranges provided in the specification and the appended claims include all values between endpoints and breakpoints.

### Definitions

"About": as used herein, the term "about", when used in reference to one or more culture conditions for cells, refers to a range of values that are similar to the reference values specified under the culture conditions. In certain embodiments, the term "about" refers to a range of values that are 25, 20, 19, 18, 17, 16, 15, 14, 13, 12, 11, 10, 9, 8, 7, 6, 5, 4, 3, 2, 1 or less percent of the reference values specified under the culture conditions.

The term "the next day" in the context refers to any time within the time range of 24 hours to 48 hours after inoculation.

"Culture medium" refers to a liquid environment in direct contact with cells in which the cells are to live, and is a collection of nutrients and buffer systems.

### Mammalian cells

As used herein, "mammalian cells" refer to mammalian-derived cell lines selected and adapted to the production conditions used in the pharmaceutical industry for the production and preparation of biological agents, and post-translational modifications of proteins the mammalian cells express have advantages in maintaining protein biological activity, stability and antigenicity. Many cell lines are available from commercial sources, such as from the American Type Culture Collection (ATCC). Non-limiting examples of mammalian cells that can be used in the present invention include the group consisting of CHO, DXB-11, DG-44, CHO/-DHFR, CV1, COS-7, HEK293, BHK, TM4, VERO, HELA, MDCK, BRL3A, W138, Hep G2, SK-Hep, MMT, TRI, MRC5, FS4, T cell lines, B cell lines, 3T3, RIN, A549, PC12, K562, PER.C6, SP2/0, NS-0, U20S, HT1080, L929, hybridomas and cancer cell lines. In at least one embodiment, the mammalian cells are CHO-S cell lines of Chinese hamster ovary cells or CHO-K1 cells of a GS deficient expression system.

### Foreign protein

As used herein, "foreign protein" refers to a protein of practical value produced by a gene encoding a target protein other than a host cell using a DNA recombination technique through effective amplification and expression of the host cell in the field of genetic engineering. The foreign protein is known in the art and may be obtained from commercial sources or by methods known in the art. In at least one embodiment, the foreign protein is an Fc fusion protein, an antibody or an enzyme; alternatively, the antibody is a monoclonal antibody or a bispecific antibody, and the monoclonal antibody may be any monoclonal antibody suitable for exogenous expression using CHO-K1 cells, including but not limited to IgG, IgA monoclonal antibodies. As used herein, "bispecific antibody" refers to an antibody having two antigen-binding sites, each of which binds to a different epitope of the same antigen or to a different epitope of a different antigen. In one embodiment, the "bispecific antibody" has binding specificities for a first antigen and a second antigen.

In at least one embodiment, the enzyme refers to an enzyme product for therapeutic drug use, i.e., a pharmaceutical enzyme, including but not limited to glucosidase, lipase, and the like.

The host cells used in the embodiment of the present invention are CHO-K1 cells, into which nucleic acid molecules encoding the ADI-31853 monoclonal antibody and the INGLP bispecific antibody (BsAb) are introduced by a conventional molecular biology means, and stably expressing cell lines are selected. The experimental equipment and the experimental reagents used in this embodiment are all available from the market, and the specific information thereof is as follows:
Experimental equipment

| Name | Manufacturer | Model |
|---|---|---|
| Cell culture bioreactor | Applikon | Ezcontrol |
| XCellTM ATF | REPLIGEN | ATF-2 |
| Clean bench (Small) | AIRTECH | SW-CJ-1FD |
| Laboratory pH meter | METTLER TOLEDO | FE20 |
| Cell counter | BECKMAN COULTER | Vi-Cell XR |
| Full-automatic biochemical analyzer | Roche | Cedex Bio HT |
| Cence centrifuge | Cence | TDZ5-WS |
| Microcentrifuge | Thermo Scientific | Microcl 17 |
| Small tube connecting machine | Fresenius Kabi | CompoDock |
| WAVE bioreactor | Sartorius | RM20 |
| High performance liquid chromatograph | Agilent | Agilent 1260 |

Reagent

| Name | Catalog No. | Manufacturer |
|---|---|---|
| Dynamis culture medium | A26175 | Gibco |
| CD CHO culture medium | 12490 | Gibco |
| Feed C+/483 culture solution | A13275-01 | Gibco |
| 200 g/L Glucose | 1.37048.5000 | Merck |

The amino acid sequences of the heavy chain (HC) and the light chain (LC) of the ADI-31853 monoclonal antibody (also called anti-LAG3 antibody) are SEQ ID NO: 1 and SEQ ID NO: 2, respectively.

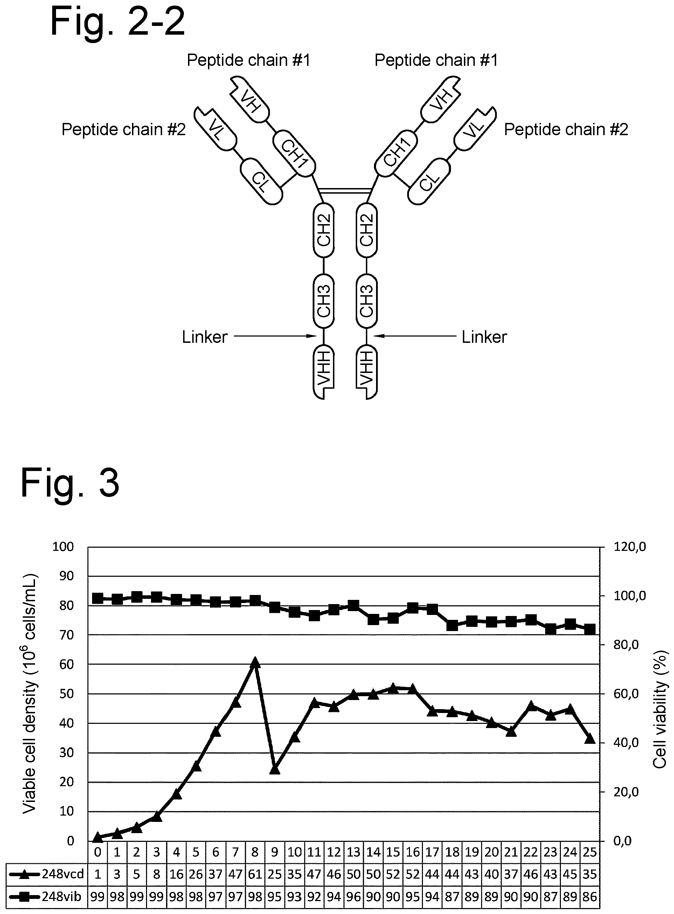

The schematic structural diagram of the INGLP bispecific antibody (also called anti-LAG3/PDL1 antibody) is shown in FIG. 2-1, wherein the antigen A is LAG-3, the antigen B is PD-L1, and the anti-LAG-3 monoclonal antibody is connected with the heavy chain variable region domain (VHH) of an anti-PD-Ll singledomain antibody (sdAb) through a flexible linker (sequence GGGGSGGGGS) to form a bilateral symmetric bispecific antibody (comprising a peptide chain #1 and a peptide chain #2 on both sides); the specific structure of the antibody is shown in FIG. 2-2, wherein the amino acid sequences of the peptide chain #1 and the peptide chain #2 are SEQ ID NO: 3 and SEQ ID NO: 4, respectively.

### Example 1: Use of Cell Culture Method Based on High-Density and Continuous Inoculation for Production of ADI-31853 Monoclonal Antibody

CHO-K1 cells stably expressing an ADI-31853 monoclonal antibody were used as test materials, and the antibody production was carried out by adopting the method described below, wherein the upstream processes of the antibody production comprise seed resuscitation, shake flask amplification, rocking reaction bag amplification, N-1 seed tank production and culture fermentation tank production. Taking the production process in a 2 L bioreactor as an example, the method was described below.

The seed cells were firstly thawed in a water bath at 37 °C, and then added into a shake flask containing a CD CHO culture medium for amplification. The culture conditions for seed resuscitation were as follows: culturing for 3 days at 36.5 °C and 130 rpm in 6% CO₂. The shake flask amplification process was as follows: inoculating at 0.5 × 10⁶ cells/mL, and the culture conditions were as follows: gradually amplifying to 12 days with one stage for every 3 days at 36.5 °C and 130 rpm in 6% CO₂.

The CHO seed cells capable of stably expressing the ADI-31853 monoclonal antibody, which had been subjected to the above-described operations, were inoculated at a cell density of about 1.0 × 10⁶ cells/mL into a 2 L Ezcontrol reactor of Applikon equipped with ATF perfusion equipment (pore size 30 kD, cell retention), wherein the culture volume was 1.2 L, and the culture medium was a Dynamis mixed 5% Feed C+/483 culture solution. The graph of the cell viability (VIB) and the viable cell density (VCD) of the CHO seed cells in an N-1 seed tank over time is shown in FIG. 3.

When the viable cell density reached more than 60 × 10⁶ cells/mL after the cells were cultured in the N-1 seed tank for 8 days, a sufficient amount (less than or equal to half the volume of the N-1 seed culture tank) of seed cell solution was taken out and inoculated according to a density of 20 × 10⁶ cells/mL in a 2 L reactor to realize high-density fed-batch culture. The first supplementation time and the temperature decrease time were simultaneously advanced from day 3 to day 1, and the temperature was decreased to 33.0 °C. Under the condition of keeping the same supplement amount as that used in the normal fed-batch culture mode, a continuous supplementation mode was adopted, namely the supplement was continuously added into the N-1 seed tank within 2 days at a certain speed, and the components of the culture medium in the N-1 seed tank were kept in a stable state. Because the inoculation density was increased by 20 times, the multiplication growth time of the inoculated cells in the culture fermentation tank could be significantly shortened, and the cells were promoted to enter a high-density stage in advance for protein expression compared with the original fed-batch process (namely, a onetime supplementation mode), so that the production time of the monoclonal antibody was shortened from 15 days of fed-batch culture to about 8 days, and the production efficiency was improved.

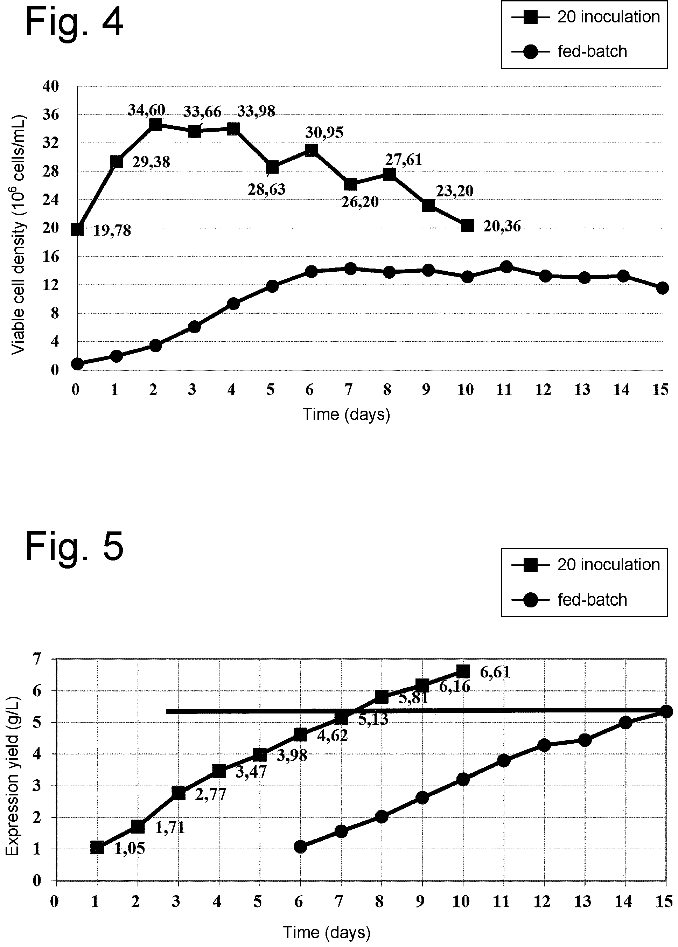

To further illustrate the advantages of the cell culture method based on high-density and continuous inoculation described in the present invention, the following experiments were also performed: inoculations in a 2 L reactor at a high density of 20 × 10⁶ cells/mL and at a density of 1 × 10⁶ cells/mL used in normal fed-batch culture; the effects of different initial inoculation densities on cell viability and viable cell density were compared, and the test results are shown in FIG. 4. The trend of the protein expression yield of CHO seed cells over time under the conditions of different inoculation densities was determined, and the specific test results are shown in FIG. 5.

It can be seen from the results in FIGs. 4 and 5 that: (1) through the ATF perfusion equipment, the CHO seed cells stably expressing the ADI-31853 monoclonal antibody could reach a high cell density, and the state of the cells could be maintained well (see FIG. 4); (2) after the high-density fed-batch culture inoculation was selected, the expression yield in the normal fed-batch culture for 15 days was realized on days 7-8, and thus the culture period was shortened (see FIG. 5).

### Example 2: Use of Cell Culture Method Based on High-Density and Continuous Inoculation for Production of INGLP Bispecific Antibody

CHO-K1 cells stably expressing an INGLP bispecific antibody were used as test materials, and the antibody production was carried out by adopting the method described below, wherein the upstream processes of the antibody production comprise seed resuscitation, shake flask amplification, rocking reaction bag amplification, N-1 seed tank production and culture fermentation tank production. Taking the production process in a 2 L bioreactor as an example, the method was described below.

Seed cells were firstly thawed in a water bath at 37 °C, and then added into a shake flask containing a CD CHO culture medium for amplification, wherein the culture conditions for seed resuscitation were as follows: culturing for 3 days at 36.5 °C and 130 rpm in 6% CO₂. The shake flask amplification process was as follows: inoculating at 0.5 × 10⁶ cells/mL, and the culture conditions were as follows: gradually amplifying to 12 days with one stage for every 3 days at 36.5 °C and 130 rpm in 6% CO₂.

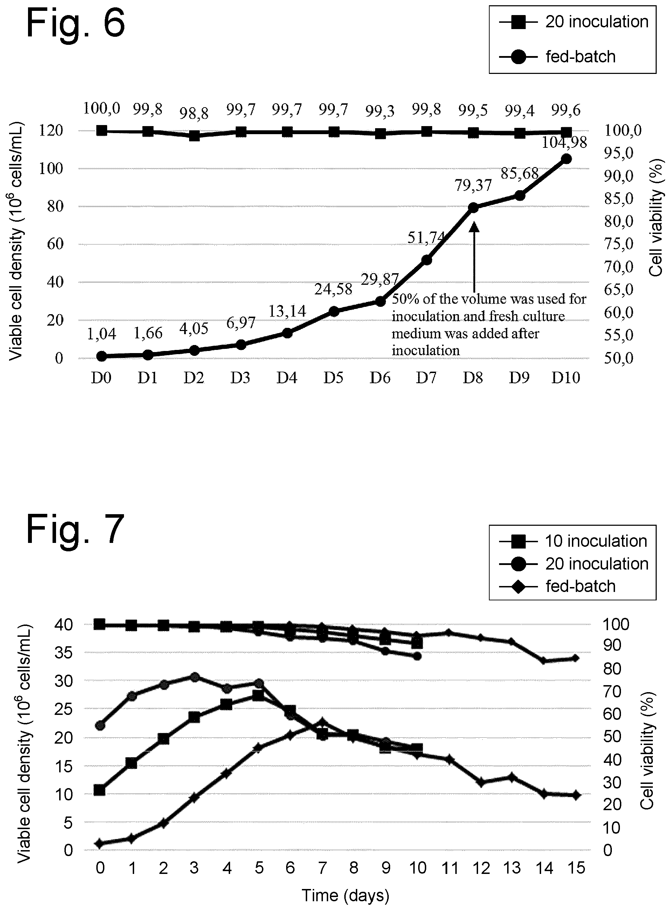

A Sartorius RM20 reactor was adopted to load a 10 L culture bag having a membrane (0.2 µm) at the bottom for culture solution filtration and cell retention, wherein the culture medium was a Dynamis mixed 5% Feed C+/483 culture solution. The CHO seed cells to be inoculated into an N-1 seed tank were cultured to have a density of about 80 × 10⁶ cells/mL. The graph of the cell viability (VIB) and the viable cell density (VCD) of the CHO seed cells in the N-1 seed tank over time is shown in FIG. 6.

It can be seen from the graph of the viable cell density in FIG. 6 that: the viable cell density in the N-1 seed tank reached79.37 × 10⁶ cells/mL at day 8 of cell culture. At this point, 5 L of the cell culture solution was taken out from the N-1 seed tank and inoculated into a culture fermentation tank, and after completion of the inoculation, the N-1 seed tank was supplemented with 5 L of a fresh culture medium for continuous cell amplification culture. On day 9 of the cell amplification culture, the viable cell density in the N-1 seed tank reached 85.68 × 10⁶ cells/mL, and the culture medium could be inoculated into another new culture fermentation tank again.

To further illustrate the advantages of the cell culture method based on high-density and continuous inoculation described in the present invention, the following experiments were also performed: when the viable cell density of the CHO seed cells in the N-1 seed tank reached about 80 × 10⁶ cells/mL, the cells were inoculated at cell densities of 10 × 10⁶ cells/mL and 20 × 10⁶ cells/mL into two 2 L Sartorius BiostatA reactors with a culture volume of 1.2 L, respectively, wherein the pre-culture temperature was 36.5 °C, and the temperature was decreased to 33.0 °C when the cell density reached about 25 × 10⁶ cells/mL. The supplementation mode was as follows: before D5 days, matching according to the integral of viable cell concentration (IVCC) to the time and the historical fed-batch culture supplement amount, and feeding every other day. About 2.8% (v/v) of the supplement was added every other day after D5 days until the yield was comparable to that at harvest in the control fed-batch culture (i.e., the normal fed-batch culture mode).

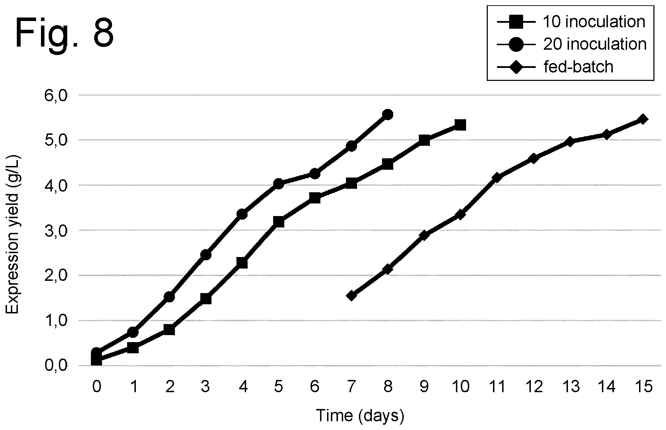

The inoculations were carried out in a 2 L Sartorius BiostatA reactor at high densities of 10 × 10⁶ cells/mL and 20 × 10⁶ cells/mL and at a density of 1 × 10⁶ cells/mL used in normal fed-batch culture, respectively, and the effects of different initial inoculation densities on cell viability and viable cell density were compared, and the specific test results are shown in FIG. 7. The trend of the protein expression field of CHO seed cells over time under the conditions of different inoculation densities was determined, and the specific test results are shown in FIG. 8.

It can be seen from the results in FIGs. 7 and 8 that: (1) through the perfusion culture in RM20 (cell retention), the CHO cells stably expressing the INGLP bispecific antibody could reach a high cell density, and the state of the cells could be maintained well (see FIG. 7); (2) after the high-density fed-batch culture inoculations were selected at 20 × 10⁶ cells/mL and 10 × 10⁶ cells/mL, the expression yield in the normal fed-batch culture for 15 days was realized on day 8 and day 10 respectively, and the culture period was shortened (see FIG. 8).

The specific embodiments of the present invention have been described above in detail. However, the above-described examples are merely exemplary examples, and the present invention is not limited to the examples described above. For those skilled in the art, any equivalent modifications and substitutions to the utility shall be within the scope of the present invention. Accordingly, without departing from the spirit and scope of the present invention, equivalent alterations and modifications shall be included within the scope of the present invention.

## Claims

1. A cell culture method based on high-density and continuous inoculation, comprising the following steps:
(1) providing a cell culture, and performing resuscitation, shake flask amplification culture and rocking reaction bag amplification culture on the cell culture;
(2) transferring the resuscitated and amplified cells into a last-stage cell amplification tank for continuous amplification culture;
(3) inoculating the cells in the last-stage cell amplification tank into a culture fermentation tank in a high-density and continuous inoculation mode for fermentation culture; and
(4) harvesting a target product.

2. The method according to claim 1, wherein the last-stage cell amplification tank in step (2) is provided with a filtration device, and non-cellular materials in the culture medium can be exchanged with a fresh culture medium by using the filtration device, so that a cell density in the last-stage cell amplification tank is more than 10⁷ cells/mL; preferably, the filtration device is ATF, Spin filter or TFF.

3. The method according to claim 1 or 2, wherein the high-density and continuous inoculation in step (3) is to inoculate the cells into the culture fermentation tank at a density of more than 10⁷ cells/mL

4. The method according to claim 3, wherein the high-density and continuous inoculation in step (3) is to inoculate a cell culture solution with a volume less than or equal to half the volume in the last-stage cell amplification tank into the culture fermentation tank at the density of more than 10⁷ cells/mL, and after the inoculation is completed, the last-stage cell amplification tank is supplemented with the same volume of fresh culture medium, so that the fresh culture medium can be inoculated into another new culture fermentation tank the next day after inoculation; the above operations of inoculating the cell culture solution into the culture fermentation tank and supplementing the fresh culture medium in the last-stage cell amplification tank are repeated.

5. The method according to any one of claims 1 to 4, wherein the resuscitation, the shake flask amplification culture and the rocking reaction bag amplification culture in step (1) are performed on the cell culture in two batches, and another last-stage cell amplification tank is added in step (2).

6. The method according to any one of claims 1 to 5, wherein a volume of the last-stage cell amplification tank in step (2) is 2 L to 1000 L.

7. Use of the method according to any one of claims 1 to 6 for the culture of mammalian cells.

8. The use according to claim 7, wherein the mammalian cells are selected from the group consisting of CHO, DXB-11, DG-44, CHO/-DHFR, CV1, COS-7, HEK293, BHK, TM4, VERO, HELA, MDCK, BRL3A, W138, Hep G2, SK-Hep, MMT, TRI, MRC5, FS4, T cell lines, B cell lines, 3T3, RIN, A549, PC12, K562, PER.C6, SP2/0, NS-0, U20S, HT1080, L929, hybridomas and cancer cell lines.

9. The use according to claim 7 or 8, wherein the mammalian cells are CHO-S or CHO-K1 cells.

10. The use according to any one of claims 7 to 9, wherein the mammalian cells comprise a nucleotide sequence encoding a foreign protein; preferably, the foreign protein is an antibody; more preferably, the antibody is a monoclonal antibody or a bispecific antibody.
